# EUROPEAN PATENT APPLICATION

(11) **EP 4 506 029 A1**
(43) Date of publication of application: **12.02.2025**
(21) Application number: 24182292.3
(22) Date of filing: 14.06.2024
(51) Int. Cl.: A61N 1/04, A61F 7/02, A61N 1/30

(54) **TERAPEUTIC SYSTEM BASED ON TRANSDERMAL TRANSMISSION OF PELOIDES, CLAYS AND DERIVATIVES AND USES THEREOF**

(30) Priority: 19.06.2023 PT 2023118737
(71) Applicant: Faculdade de Medicina Veterinária da Universidade De Lisboa, 1300-477 Lisbon (PT)
(72) Inventor: TILLEY, PAULA, 2580-319 ALENQUER (PT); BASTOS, CARLA, 3800-373 AVEIRO (PT); GOMES, NUNO, 3800-373 AVEIRO (PT); ROCHA, FERNANDO, 3810-489 AVEIRO (PT); BARROSO, LIA, 4910-649 VILE (PT)
(74) Representative: Gata-Goncalves, Ligia

(57) **Abstract**

The present invention relates to a therapeutic system for use in the treatment and prevention of tendon and musculoskeletal lesions.

The therapeutic system of the invention combines electropelotherapy to deliver active principles to an animal by transdermal transmission under heating conditions, said active principles being contained in clays and peloids.

The system comprises 2 main elements, i) an electronic controller unit A and ii) a delivery unit B comprising a patch with the active principles to be applied transdermally by iontophoresis under heating to the body of an animal.

The present invention is in the technical field of medical devices in particular of medical devices to be used in medical rehabilitation or prevention of tendon and musculoskeletal injuries.

## Description

### Technical domain of the invention

The present invention relates to a therapeutic system for use in the treatment and prevention of tendon and musculoskeletal lesions.

The therapeutic system of the invention combines electropelotherapy to deliver active principles to an animal by transdermal transmission under heating conditions, said active principles being contained in clays and peloids.

The system comprises 2 main elements, i) an electronic controller unit A and ii) a delivery unit B comprising a patch with the active principles to be applied transdermally by iontophoresis under heating to the body of an animal.

The present invention is in the technical field of medical devices in particular of medical devices to be used in medical rehabilitation or prevention of tendon and musculoskeletal injuries.

### Prior art

The therapeutic protocols applied to physical medicine and rehabilitation (PMR) involve a large spectrum of therapeutic modalities, including hydrotherapy, balneotherapy, pelotherapy, physiotherapy and physical exercise. These classical practices are based on the experience of the baths in medicinal waters, on the ingestion of mineral-medicinal water, on mud baths, or on applying cataplasms with therapeutic clays on specific areas of the body.

PMR, medical hydrology and physiotherapy are all medicine fields that use specific methods for the rehabilitation, therapy and prevention of various diseases, as well as for the follow up of ambulatory and in hospital treatments of certain chronic diseases that require rehabilitation methods.

From ancient times, humanity has empirically used clays, muds or clayey soils for therapeutic purposes, through ingestion or topical application as cataplasms or mud baths. Various traditional veterinary practices use zootherapeutic resources in the health care of domestic animals, where medicinal value maintains their relevance in ethnoveterinary medicine, the scientific designation for the traditional care in animal health.

The potential benefit in the use of therapeutic clays on equine lesions leading to lameness is to minimize the secondary effects associated to the systemic, topical or intra-articular administration of anti-inflammatory medicines, as well as sustaining a local delivery of therapeutical elements.

Clays are commonly included in pharmaceutical formulations, cosmetic treatments, spas and in aesthetic medicine, as active ingredients or as excipients. The absorption properties of clays have been explored in a variety of cosmetic and pharmaceutical formulations as a contribution to the cure of certain diseases. Furthermore, their capacity for cationic exchange and their extremely fine particle size, are relevant characteristics in the removal of oils, secretions, toxins and skin contaminants. Experiments to evaluate cationic exchange have shown that the antibacterial component of clays can be removed, implying the presence of permutable cations in the antibacterial process.

When introduced as excipients in therapeutic formulations, clays improve the organoleptic characteristics of the formulations, such as flavour, smell and colour, or their physical and chemical properties, like viscosity for example. They may also be used as an adjuvant, facilitating the preparation of the formulations and they may on the other hand promote the disintegration of the formulation when administered orally.

The therapeutic action of clay as an active ingredient is used in pharmaceutical formulations administered orally as gastrointestinal protectants, laxatives or antidiarrheals, but also in topical uses as dermal protectants or cosmetics. Their use in spas is related to the therapeutic activity of the clay minerals. In aesthetic medicine they are used in skin cleansing and to fight lipodystrophies like acne and cellulitis.

Pelotherapy is a passive non-invasive technique involving applying hot clays, designated peloids, with therapeutic objectives. The heated clays, when in contact with the skin, promote several reactions which are responsible for the therapeutic effect of the essential elements of the clay. Various products with a clay base, peloids, have a traditional therapeutic history, as they are easily available in nature for human use or are available on the market as 100% natural products.

In order to have a good interaction between the peloid and the skin it is necessary to comply with some requirements, which refer to a greater release of ionizable elements and their elimination for percutaneous absorption. Ideally, the results should be physiologically efficient in a passive percutaneous absorption.

The capacity for cationic exchange (CEC) and the other characteristics of the formulation may define the efficiency in percutaneous deepness achieved by the ions, as well as their desirable absorption by the skin. The success of a complete percutaneous action may be observed through pain relief, anti-inflammatory effect, improvement of the range of the movement of the upper limbs, antibacterial effect, healing effect, amongst others.

The main objective in pelotherapy is to reduce pain, alleviate muscle spasms, improve mobility and muscle tone. Several studies, carried out in thermal and thalassotherapy centres, involving the use of peloids in rehabilitation programs, have shown excellent results in the treatment of skin and musculoskeletal diseases.

Electrotherapy is the most often used therapeutic modality in PMR and in physiotherapy, where the use of the iontophoresis technique for transdermal administration of medicines is considered to have numerous theoretical advantages when compared to other forms of drug administration.

The main advantage is the fact of being a non-invasive technique which increases skin permeability to substances carrying an ionic charge, improving therapeutic efficiency, avoiding the metabolism and preventing the inconveniences caused by parenteral or oral drug administrations which need to be absorbed by the systemic circulation, reducing this way the possibility of dose variations. It has been demonstrated that the possibility of "programming" an administration of the substances which are ionically charged enables a therapeutic framework which increases patient adherence.

Clays are naturally ionized, and it is possible to change their polarity by using electrolytic solutions or by mixing them with specific medicinal-mineral water (maturation process).

In pelotherapy the therapeutic action of peloids is achieved in a localized as well as a systemic way through two physiologic penetration pathways, through the pores, sweat glands, sebaceous glands and hair follicles, and by diffusion though the *stratum corneum.* Nevertheless, the action of peloids is limited by the excellent barrier function exerted by the *stratum corneum,* the same way as in the transdermic administration of drugs like diclofenac, corticosteroids or steroids.

**Table 1. Identification of the converging points between the two therapeutic modalities**

| **Application / uses** | **Pelotherapy** | **Electrotherapy (Iontophoresis)** |
|---|---|---|
| **Health Area** | Medical Hydrology | Physical Medicine and Rehabilitation |
| **Treatment vehicle** | Skin | Skin |
| **Type of treatment** | Non-invasive | Non-invasive |
| **Therapeutic substance** | Medicinal or cosmetic peloid | Drugs and cosmetics |
| **Transdermal delivery** | Passive | Active |
| **Energy source** | Heat | TENS¹ |
| **Localized physiological phenomena** | Vasodilation | Vasodilation |
| | Local hyperaemia | Local hyperaemia |
| | | Local analgesia |
| | Local analgesia | |
| **Average reference treatment time** | 20-45 min | 20-45 min |
| **Most common treatments** | Rheumatism | Rheumatism |
| | Musculoskeletal pain syndromes | Musculoskeletal pain syndromes |
| | Dermatosis | Dermatosis |
| **Local and systemic effect** | Anti-inflammatory | Anti-inflammatory |
| | Increased muscle tone | Increased muscle tone |
| | Analgesic | Analgesic |
| | Wound healing | Wound healing |
| **Scientific reports** | Empirical evidence | Experimental evidence |

The mechanisms involved in the iontophoretic transport are electromigration, the movement of ions through a membrane (skin)by the direct action of an electrical field, and electroosmosis, the volume of flux induced by the current.

The pre-requirements to manage the delivery include a good electrical conductivity, good mechanic properties, good bio-adherence and acceptable viscoelastic properties, to assure clinical efficiency and conformity on the patient.

The transdermal transmission in pelotherapy using temperature and electricity via iontophoresis, was tested by Szántó et *al.* (1998) in diffusion cells *in vitro* (Szántó, Z.; Papp, L. Effect of the different factors on the iontophoretic delivery of calcium ions from bentonite. J. Control Release 1998, 56, 239-247). This document discloses the use of a "Franz cell diffusion" device providing heating and pulsated current to enable the permeation and diffusion *in vitro* tests using bentonite previously enriched with calcium as peloid, and biological membranes, which in this case was pig skin.

The challenge of pelotherapy and of electrotherapy (iontophoresis) as individual therapeutic methods is the crossing of some ionic species. The use of iontophoresis on peloids has shown to be relevant since, through an electrical potential (tension) between the peloid and the skin, the transfer of ions from the elements that are present in the peloid is accelerated. The aforesaid confers on this method usefulness in attaining an efficient transfer of the essential elements of the peloid as an analgesic, antiseptic or anti-inflammatory agent.

None of the prior art techniques as above-mentioned, and when applied with therapeutic objectives, promote enough interaction between the peloid and the skin of the injured animal, thus promoting healing effect by pain relief, anti-inflammatory effect, antibacterial effect amongst others.

The present invention provides a solution for the mentioned problem by developing a therapeutic system that combines pelotherapy and of electrotherapy (iontophoresis) under heating conditions to deliver the active principles contained in the peloids, clays and derivatives thereof by creating the optimal conditions for improving the ions transference through the skin of an animal.

### Description of the figures

The figures herein presented refer to specific embodiments of the therapeutic system of the invention.

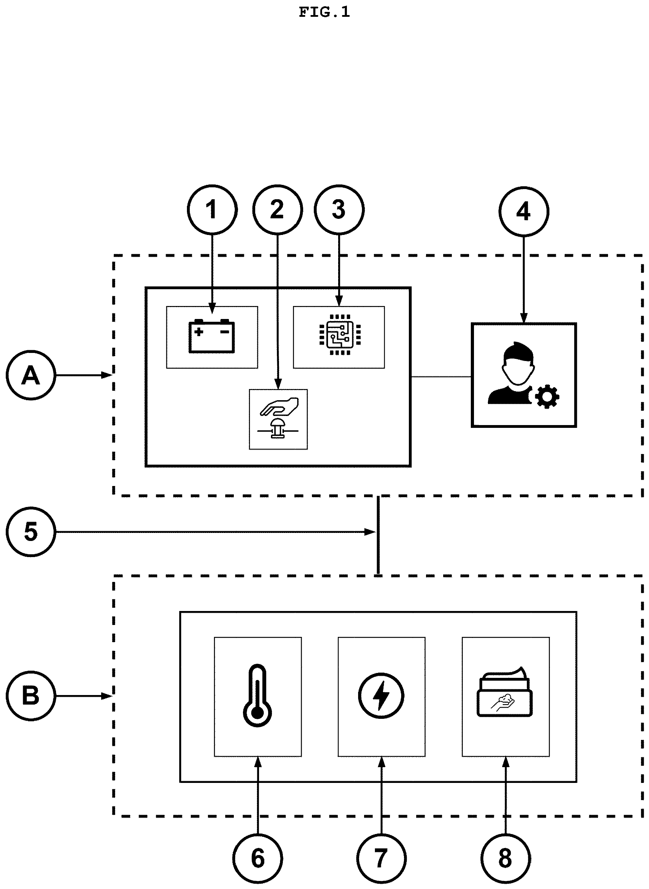

**Fig. 1****.** Shows a representation of the therapeutic system according to the present invention comprising 2 units, **controller unit A** and **delivery unit B,** wherein:
   **Unit A comprises:**
      1. A power source, ex. rechargeable battery,
      2. Emergency stop button,
      3. Control and monitoring electronic system,
      4. Human-Machine Interface,
      5. Electrical connection between A and B, and
   **Unit B comprises:**
      6. Heater sensors,
      7. Iontophoresis electrodes,
      8. Container for the active principles.
   The electronic controller (e-controller) of **Unit A** enables the configuration of the treatment temperature (°C) and electric current intensity (mA), as well as its intended duration (minutes), by pressing the "start" button. It is also possible to store the selected treatment parameters for future use, by operating the "program selection" button.

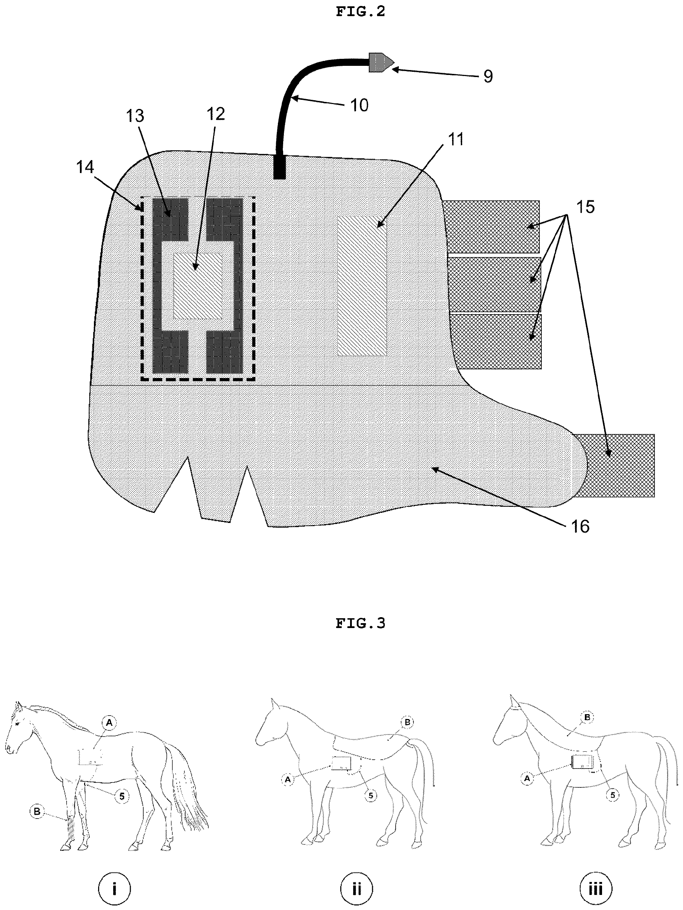

**Fig. 2** Represents a detail of the **unit B,** in a preferred embodiment of the invention, wherein:
   9. Electrical interface for connecting a cable to A,
   10. Electrical cable,
   11. Iontophoresis electrode (cathode/anode),
   12. Iontophoresis electrode (anode/cathode),
   13. Active heating area,
   14. Pocket for placing a patch with the active principles,
   15. Fixing system, ex Velcro stripes,
   16. Laminated fabric support.
**Fig. 3** Represents a preferred embodiment of the therapeutic system of the invention showing **unit A** connected with **unit B** via cable connection **5,** wherein:
   - **unit A** and **unit B** are placed on the body of a horse, and
   - **unit B** is a laminated fabric support **16** forming a sleeve having a fixing system **15** with Velcro **or** a textile rug placed on:
      (i) a horse limb (fore or hind limb), wherein **unit B** is a sleeve fixed to the limb of a horse by Velcro system,
      (ii) on the back of a horse, wherein **unit B** is a textile rug,
      (iii) a textile rug applied on the neck of a horse, wherein **unit B** is also a textile rug.

### Description of the invention

The present invention relates to a therapeutic system for use in the treatment and prevention of tendon and musculoskeletal lesions.

The system of the invention combines electropelotherapy to deliver active principles to an animal by transdermal transmission under heating of said active principles, those being clays, peloids or mixtures thereof.

Therefore, the therapeutic system comprises 2 main elements: **an electronic controller unit A** and a **delivery unit B,** for delivering the active principles, hereafter also called as peloids to be applied transdermally.

**Unit A** is an electronic controller comprising an electronic application capable of being programmed to set the temperature, time and electrical current of **unit B.**

In a preferred embodiment, unit A comprises a network connection, preferably a Wi-Fi connection.

Unit A further comprises ports for connecting to an electric source, a screen to display and set the programable parameters of the treatment and an ON/OFF knob (not shown in the figures).

The screen of unit A is able to display the different parameters of the therapeutic system of the invention. The screen can also display the current parameters of the treatment further allowing to pause, resume and cancel the ongoing treatment. The screen of unit A can be a touch screen activated by a finger or by a specific electronic pen. In a preferred embodiment, the screen is a touch screen.

Unit A can also comprise an emergency stop button **2** to stop the treatment without the need of accessing the screen, i.e., for a rapid shutting down the system.

In a preferred embodiment, unit A is able to set different programs with combinations of temperature, time and electrical current of unit B.

In another embodiment the screen displays the current parameters of the treatment along with the skin temperature of the subject.

Unit A can also comprise a sound system allowing to signalise the end of the treatment and/or an emergency situation, such as a malfunction of the unit, an error in the selected program or other security features of unit B, such as overheating or exceeding the programmed time.

The electric power can be provided to the unit A by a charger, a battery or via electrical main. In a preferred embodiment, the source of electric power is a rechargeable battery **1.**

**Unit B** is a laminated fabric support device **16** having a recipient or pocket **14** for accommodating the active principles **8** and the active heating system, thus working as direct interface for the treatment in the animal body. The support **16** is ergonomically adapted to the region to be treated and includes the electrical circuits and sensors.

Unit B further comprises the circuit to connect the interface cable **9** to the heating element based on a resistive material, two flexible conductive surfaces (sensors) to induce the iontophoresis current and at least two temperature sensors for control and monitoring.

In the scope of the present invention, an adequate peloid possesses several essential properties that contribute to its therapeutic value, such as Terdax^{®} (France), Ghassoul (Morocco), Euganean Thermal muds (Italy), Copahue (Argentina), Dona Beija (Azores, Portugal) and other natural and enhanced peloids with recognized and validated therapeutic effects through clinical trials.

In a preferred embodiment, the peloids are the ones used for their rheumatic therapeutic action comprising high sulphur concentration. In a further preferred embodiment, the peloids present anti-inflammatory properties and therapeutic effect to alleviate rheumatic conditions and muscle-skeletal disorders.

The system of the invention is capable of applying a temperature of 30 to 50°C, preferably of 35 to 45°C to unit B. Typically, temperatures above 50°C can modify the therapeutic properties of the peloids, which may behave like an inert material, not being adequate for the therapeutic purposes of the invention.

The system of the invention is capable of applying a current to unit B with an intensity of 0.1 to 5.0 mA, preferably of 0.05 to 4.5 mA, more preferably of 1.0 to 4.0 mA, even more preferably of 1.0 to 4.0 mA, in particular the intensity of the applied current is of 1.5 to 3.5.

The fabric support **16** of unit B can be a laminated complex, that perfectly adapts to the animal body by means of the shape and can comprise a foam. In one preferred embodiment fabric support **16** comprises 3 main parts:
i. An external layer, based on a carded fabric that helps on the final fixation to the subject,
ii. An intermediate layer comprising a viscoelastic foam, and
iii. An inner layer comprising a technical fabric with hydrophobic and breathable properties placed directly in contact with the subject. This layer can function as an encapsulant for the electrical circuit. The conductive iontophoresis electrodes are only exposed in strategic defined areas to comply with the treatment.

The activation of the transdermic debit by electrokinetic forces applies a current up to 0,5 mA/cm² as above defined, a temperature between 40 and 45 °C, also as above defined, and a duration of application of 10 to 30 minutes, preferably a duration of 15 to 20 minutes.

## Claims

1. A therapeutic system for use in the treatment and prevention of tendon and musculoskeletal lesions of an animal, **characterized by** comprising 2 separated units, an electronic controller unit A and a delivery unit B, wherein:
- the **controller unit A** assembly comprises an electronic system 3 for controlling and monitoring a designed application (APP), a human-machine interface **4,** means of providing electric power **1** and means of connection **5** to the unit B, and
- the **delivery unit B** is a laminated fabric support **16** comprising a heating element **6** of a resistive material, comprising temperature sensors, iontophoresis element **7** comprising the electrodes, a pocket **14** for placing a patch with the peloid or a mixture thereof **8,** means of fixation **15** of unit B to the body of an animal, and means of connection **5** to the unit A,
wherein:
- the designed application (APP) of unit A is programable to set the temperature, time and electrical current of unit B,
- the peloid or a mixture thereof **8** of unit B is delivered transdermally to the body of said animal by iontophoresis under heating conditions with a temperature of 30 to 50°C, and a current with an intensity of 0.1 to 5.0 mA.

2. A therapeutic system according to claim 1 **characterized by** the unit A further comprising a network connection, preferably a Wi-Fi connection.

3. A therapeutic system according to claim 1 or 2, **characterized by** the designed application (APP) of unit A comprising the functional features of the current parameters of the treatment further allowing to pause, resume and cancel the ongoing treatment and one or more programs with combinations of temperature, time and electrical current to deliver to unit B.

4. A therapeutic system according to claim 1 to 3, **characterized by** the screen of unit A being a touch screen.

5. A therapeutic system according to claim 1 to 4 **characterized by** the unit A further comprising an emergency stop button **2** and/or a rechargeable battery **1** as source of electric power.

6. A therapeutic system according to claim 1 to 5 **characterized by** the unit A further comprising a sound system to signalise the end of the treatment and/or an emergency situation, preferably the malfunction of the unit, an error in the selected program or other security features of unit B, preferably as overheating or exceeding the programmed time.

7. A therapeutic system according to claim 1 **characterized by** the laminated fabric support **16** of unit B comprising 3 mains layers:
i. An external layer based on a carded fabric having at one its extremity fixation means or part to allow its fixation to the animal body,
ii. An intermediate layer comprising a viscoelastic foam, and
iii. An inner layer comprising a functional fabric with hydrophobic and breathable properties placed directly in contact with the body of the animal.

8. A therapeutic system according to claim 7 **characterized by** the laminated fabric support **16** of unit B defining a sleeve **or** a textile rug having a Velcro system as fixing system **15.**

9. A therapeutic system according to claim 7 or 8 **characterized by** the laminated fabric support **16** of unit B further comprising a pocket for accommodating the unit A.

10. A therapeutic system according to claim 1 **characterized by** the peloid or a mixture thereof **8** being Terdax^{®}, Ghassoul, Euganean Thermal muds, Copahue, and/or Dona Beija.

11. A therapeutic system according to any of the previous claims **characterized by** the temperature applied to unit B is of 35 to 45°C.

12. A therapeutic system according to claims 1 to 10 **characterized by** the intensity of the current applied to unit B is of 0.05 to 4.5 mA, preferably of 1.0 to 4.0 mA, more preferably of 1.0 to 4.0 mA, even more preferably the intensity of the applied current is of 1.5 to 3.5 mA.

13. A therapeutic system according to any of the previous claims **characterized by** being specifically developed to be placed on a horse.
